# EUROPEAN PATENT APPLICATION

(11) **EP 4 796 056 A1**
(43) Date of publication of application: **26.08.2026**
(21) Application number: 24880151.6
(22) Date of filing: 17.10.2024
(51) Int. Cl.: A61B 17/22

(54) **STEERABLE MEDICAL INSTRUMENT**

(30) Priority: 18.10.2023 KR 20230139422; 11.10.2024 KR 20240138299
(71) Applicant: Korea University Research and Business Foundation, Seoul 02841 (KR)
(72) Inventor: YU, Cheol Woong, Seoul 06574 (KR)
(74) Representative: Biallo, Dario
(86) International application number: PCT/KR2024/015796
(87) International publication number: WO 2025/084814

(57) **Abstract**

A steerable medical instrument according to an embodiment, which is capable of moving along a blood vessel, comprises: a guide wire having an end formed in a loop shape; and a micro-conduit formed as a hollow tube and coupled to surround the guide wire. The guide wire can be inserted into or withdrawn from the micro-conduit, and the micro-conduit can rotate within the blood vessel to control the direction of movement of the guide wire.

## Description

### TECHNICAL FIELD

A steerable medical instrument is disclosed.

### BACKGROUND ART

Vascular diseases, particularly peripheral artery disease (PAD), pose serious problems for patients worldwide. These conditions involve the accumulation of plaque or calcification in the vascular inner wall, which obstructs blood flow and, in severe cases, leads to chronic total occlusions (CTO) in which the vessel becomes completely blocked. Such occlusions primarily occur in the cardiovascular system and peripheral arteries, and reopening occluded vessels requires a very high level of technical skill from the operator.

Conventional percutaneous procedures for treating CTO lesions generally involve advancing a guide wire through an occluded segment, followed by balloon angioplasty or stent implantation. In these procedures, the selection of the guide wire and precise directional control are critical. The guide wire needs to accurately follow the path within the vessel intended by an operator and pass through the lesion without damaging the vessel wall. However, existing guide wires often make directional control difficult, and in some cases, the guide wires may exit the vessel or enter the subintimal space.

This problem is especially pronounced when lesions are heavily calcified or long. Conventional guide wires have limitations in passing through such lesions, often requiring the operator to switch between multiple types of wires, which is cumbersome. Furthermore, if the guide wire damages the vessel wall during the procedure, complications such as hematoma or vessel perforation may occur. Therefore, there is an ongoing need for new methods and devices that reduce these risks.

Current CTO treatment techniques still require significant improvements in terms of guide wire directional control and procedural safety to ensure safe lesion penetration and increase procedural success rates. To address this, a variety of devices and methods that allow more precise intravascular control have recently been developed.

The above description has been possessed or acquired by the inventor(s) in the course of conceiving the present disclosure and is not necessarily an art publicly known before the present application is filed.

Prior Document: U.S. Patent Publication No. US 2021/0186726 A1 (published on June 24, 2021)

### DISCLOSURE OF THE INVENTION

### TECHNICAL GOALS

An aspect according to an embodiment is to provide a steerable medical instrument in which a guide wire is combined with a microcatheter, wherein the end of the guide wire has a J-shaped curve, allowing precise directional control within a blood vessel. This enables an operator to easily steer the guide wire in the desired direction and safely advance through a lesion without penetrating the wall of the blood vessel.

An aspect according to an embodiment is to provide a steerable medical instrument in which a guide wire is formed of a shape memory alloy, allowing flexibility and stiffness to be appropriately adjusted. This ensures that the guide wire does not deform or twist within a blood vessel even when passing through a hard, calcified lesion and may operate effectively in a long and complex lesion.

An aspect according to an embodiment is to provide a steerable medical instrument in which an assembly of a guide wire and a microcatheter is designed to safely pass through a lesion without damaging the wall of a blood vessel, thereby significantly reducing the risk of vessel perforation or hematoma formation during a procedure and improving procedural safety.

An aspect according to an embodiment is to provide a steerable medical instrument capable of handling not only short lesions but also various types of lesions, including curved, calcified, and long occluded lesions, thereby greatly enhancing procedural versatility.

An aspect according to an embodiment is to provide a steerable medical instrument that increases the success rate of recanalization of a chronic occlusive lesion of a peripheral artery, reduces an operator's burden, and enhances patient safety.

The technical aspects obtainable from the present disclosure are non-limited by the above-mentioned technical aspects, and other unmentioned technical aspects can be clearly understood from the following description by those having ordinary skill in the technical field to which the present disclosure pertains.

### TECHNICAL SOLUTIONS

A steerable medical instrument according to an embodiment for achieving the aspects includes a guide wire having an end formed in a loop shape and a microcatheter formed as a hollow tube and coupled to surround the guide wire, wherein the guide wire is able to be retracted into or extended out of the microcatheter, and the microcatheter rotates within a blood vessel to control a traveling direction of the guide wire.

According to an aspect, the guide wire may be formed of a material having a shape memory property, and the end of the guide wire may be restored to a J-shaped form within a blood vessel.

According to an aspect, the end of the guide wire may have stiffness, and the stiffness of the end may be greater than that of an occluded lesion of a blood vessel and less than that of a wall of the blood vessel.

According to an aspect, the microcatheter may include a first region including a tip through which the end of the guide wire is retracted or extended and a second region connected to the first region to form an angle with respect to the first region, and a direction in which the guide wire is extended may be controlled according to a direction of the first region.

According to an aspect, the first region may be formed in a conical or tapered shape having a diameter decreasing toward the tip, and the tip may engage with the guide wire.

According to an aspect, an outer surface or an inner surface of the microcatheter may be coated with a hydrophilic coating.

A method of controlling a steerable medical instrument formed by coupling of a guide wire and a microcatheter, wherein the guide wire has an end formed in a loop shape and the microcatheter is formed as a hollow tube, according to an embodiment for achieving the aspects, includes providing, as a real-time image, a blood vessel image captured using intravascular ultrasound (IVUS) or fluoroscopy, inserting the steerable medical instrument into a blood vessel that is occluded, extending the end of the guide wire out of the microcatheter into the blood vessel such that the end of the guide wire deforms into a loop shape, and advancing the microcatheter along the blood vessel while controlling a direction of the guide wire based on the blood vessel image.

According to an aspect, the method further includes, after the advancing of the microcatheter along the blood vessel while controlling the direction of the guide wire based on the blood vessel image, when the end of the guide wire reaches an occlusion within the blood vessel, retracting the end of the guide wire into the microcatheter, so that the end of the guide wire, in an expanded form, fractures the occlusion.

### EFFECTS OF THE INVENTION

According to a steerable medical instrument according to an embodiment, a guide wire may be coupled to a microcatheter, wherein the end of the guide wire has a J-shaped curve, enabling precise directional control within a blood vessel. This may allow an operator to easily steer the guide wire in the desired direction and safely advance through a lesion without penetrating the wall of the blood vessel.

According to a steerable medical instrument according to an embodiment, a guide wire may be formed of a shape memory alloy, allowing flexibility and stiffness to be appropriately adjusted. This may ensure that the guide wire does not deform or twist within a blood vessel even when passing through a hard, calcified lesion, and may operate effectively in a long and complex lesion.

According a steerable medical instrument according to an embodiment, an assembly of a guide wire and a microcatheter may be designed to safely pass through a lesion without damaging the wall of a blood vessel, thereby significantly reducing the risk of vessel perforation or hematoma formation during a procedure and enhancing procedural safety.

According to a steerable medical instrument according to an embodiment, it may be possible to handle not only short lesions but also various types of lesions, including curved, calcified, and long occluded lesions, thereby greatly improving procedural versatility.

According to a steerable medical instrument according to an embodiment, it may be possible to increase the success rate of recanalization of a chronic occlusive lesion of a peripheral artery, reduce an operator's burden, and enhance patient safety.

The effects of the steerable medical instrument, according to an embodiment, are not limited to the foregoing effects, and unstated effects may be clearly understood by those skilled in the art from the description below.

### BRIEF DESCRIPTION OF DRAWINGS

FIG. 1 illustrates a steerable medical instrument according to an embodiment.
FIG. 2 illustrates a steerable medical instrument with various thicknesses according to an embodiment.
FIG. 3 illustrates a microcatheter of a steerable medical instrument according to an embodiment.
FIG. 4 is a flowchart illustrating a method of controlling a steerable medical instrument according to an embodiment.
FIG. 5 illustrates a method of controlling a steerable medical instrument according to an embodiment.
FIG. 6 illustrates a method of controlling a steerable medical instrument according to an embodiment.

The accompanying drawings illustrate desired embodiments of the present disclosure and are provided together with the detailed description for better understanding of the technical idea of the present disclosure. Therefore, the present disclosure should not be construed as being limited to the embodiments set forth in the drawings.

### BEST MODE FOR CARRYING OUT THE INVENTION

Hereinafter, embodiments will be described in detail with reference to the illustrative drawings. Regarding the reference numerals assigned to the components in the drawings, it should be noted that the same components will be designated by the same reference numerals, wherever possible, even though they are shown in different drawings. Also, in the description of the embodiments, a detailed description of well-known related structures or functions will be omitted when it is deemed that such description will cause ambiguous interpretation of the present disclosure.

In addition, the terms first, second, A, B, (a), and (b) may be used to describe components of the embodiments. These terms are used only for the purpose of discriminating one component from another component, and the nature, the sequences, or the orders of the components are not limited by the terms. When one component is described as being "connected," "coupled," or "attached" to another component, it should be understood that one component may be connected or attached directly to another component, and an intervening component may also be "connected," "coupled," or "attached" to the components.

Components included in an embodiment and components that have common functions will be described using the same names in other embodiments. Unless stated otherwise, the description of any one embodiment may be applied to other examples, and the specific description of the repeated configuration will be omitted.

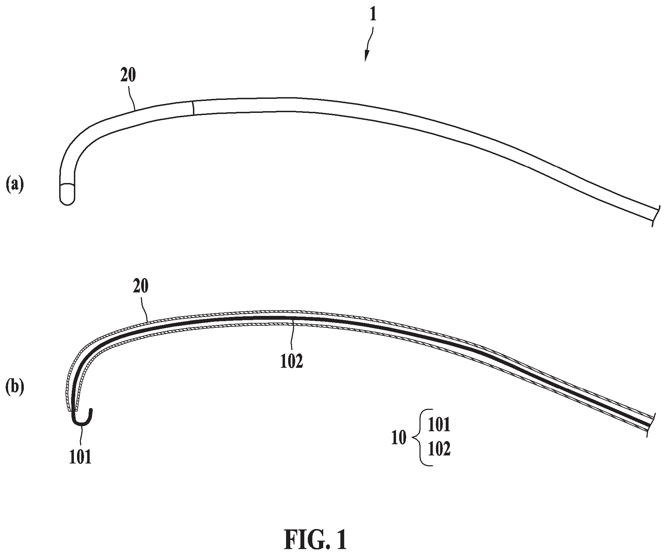

FIG. 1 illustrates a steerable medical instrument 1 according to an embodiment.

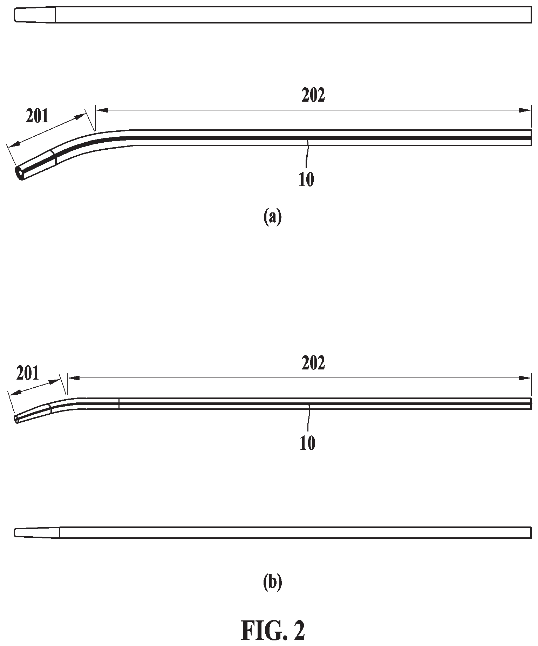

FIG. 2 illustrates the steerable medical instrument 1 with various thicknesses according to an embodiment.

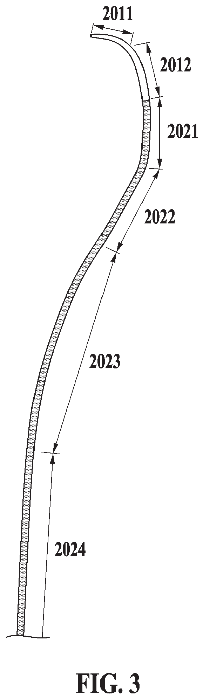

FIG. 3 illustrates a microcatheter 20 of the steerable medical instrument 1 according to an embodiment.

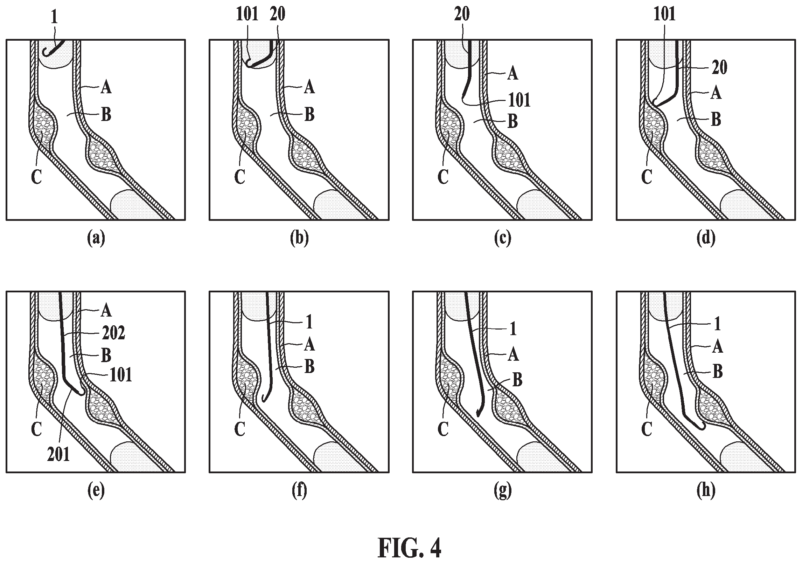

FIG. 4 is a flowchart illustrating a method of controlling the steerable medical instrument 1 according to an embodiment.

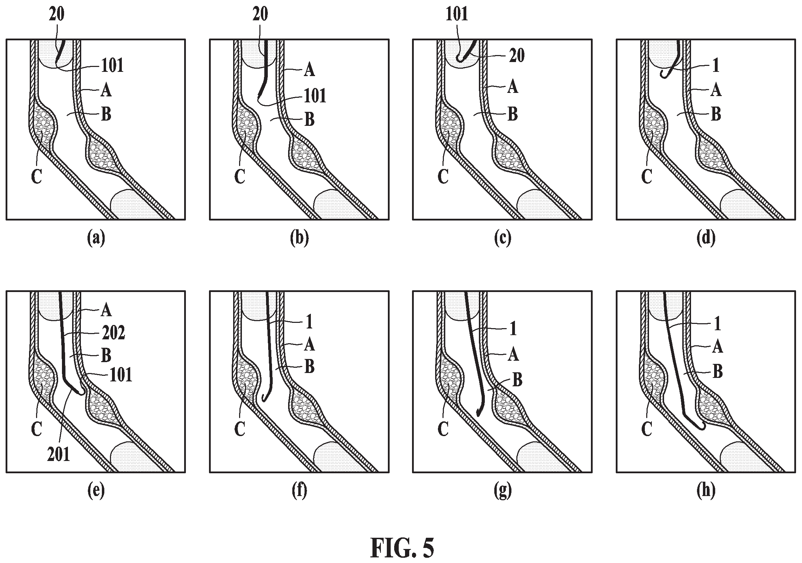

FIG. 5 illustrates a method of controlling the steerable medical instrument 1 according to an embodiment.

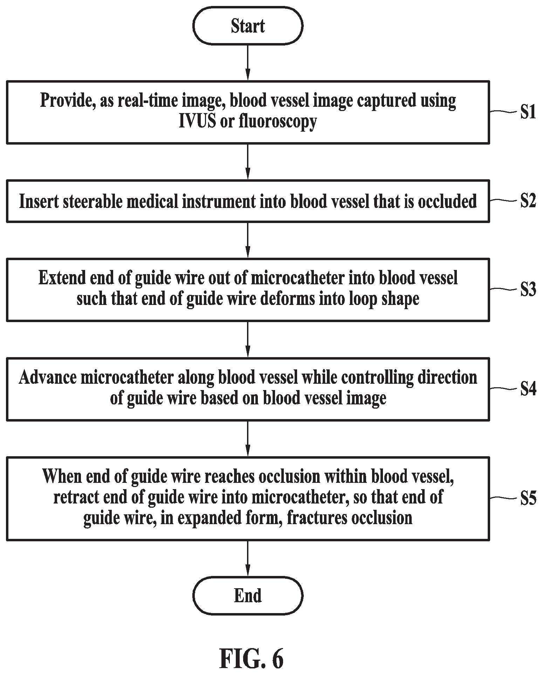

FIG. 6 illustrates a method of controlling the steerable medical instrument 1 according to an embodiment.

Referring to FIG. 1, the steerable medical instrument 1 according to an embodiment is a medical instrument 1 that may control a direction within a blood vessel and may include a guide wire 10 and the microcatheter 20.

FIG. 1(a) illustrates the exterior of the steerable medical instrument 1 according to an embodiment, and FIG. 1(b) is a cross-sectional view illustrating the interior of the steerable medical instrument 1 according to an embodiment.

The guide wire 10 may be designed to pass through a lesion without damaging the wall of a blood vessel. The guide wire 10 may be inserted into the microcatheter 20. The guide wire 10 may move longitudinally within the microcatheter 20. In this case, an end 101 of the guide wire 10 may be extended out of the microcatheter 20. Additionally, the end 101 may be retracted into the microcatheter 20. A main body 102 of the microcatheter 20 may move within the microcatheter 20.

For example, the end 101 of the guide wire 10 may be formed in a loop shape, and the main body 102 may extend from the end 101 to the end portion of the microcatheter 20. In this case, the end 101 of the guide wire 10 may maintain a straight, expanded form within the microcatheter 20 so as to extend in the same direction as the main body 102 and may deform into a loop shape when extended out of the microcatheter 20.

For example, the guide wire 10 may be formed of a shape memory alloy. For example, at least the end 101 of the guide wire 10 may be formed of a shape memory alloy. The end 101 of the guide wire 10 may be restored to a J-shaped form 21 within a blood vessel A.

As described above, the guide wire 10 formed of a shape memory alloy may be restored to the J-shaped form 21 within the blood vessel A, thereby moving flexibly even within a blood vessel having a complex curved occluded lesion B and a calcification C.

Furthermore, at least a portion of the guide wire 10 may be reinforced to pass through a hard lesion including a calcified lesion. For example, at least the end 101 of the guide wire 10 may be formed of a material having stiffness. In this case, the stiffness of the end 101 may be greater than that of the occluded lesion B but may be set less than that of the wall of the blood vessel A. Accordingly, the guide wire 10 may move through the blood vessel A while minimizing damage to the blood vessel A caused by the guide wire 10, even when passing through an occlusion.

Referring to FIG. 2, the guide wire 10 may be provided in various profiles. For example, the guide wire 10 may be provided in various thicknesses. FIG. 2(a) illustrates the steerable medical instrument 1 equipped with the guide wire 10 having a diameter of 0.018 inches, and FIG. 2(b) illustrates the steerable medical instrument 1 equipped with the guide wire 10 having a diameter of 0.014 inches. In addition, the steerable medical instrument 1 according to an embodiment may use the guide wire 10 having a diameter less or greater than that of the illustrated guide wire 10, under a condition in which the occluded lesion B may be fractured.

Referring again to FIG. 1, the microcatheter 20 may be formed as a hollow tube. The microcatheter 20 may be designed to surround the guide wire 10. That is, the guide wire 10 may be inserted into the microcatheter 20. The guide wire 10 may be retracted into and extended out of the microcatheter 20. For example, the guide wire 10 may be extended out of the microcatheter 20 into a blood vessel or retracted into the microcatheter 20. The guide wire 10 may easily move toward the occluded lesion B to be fractured along the blood vessel A by means of the microcatheter 20.

For example, a hydrophilic coating 34 may be applied to the outer surface of the microcatheter 20. Accordingly, the microcatheter 20 may advance smoothly within the blood vessel A, so that an operator may easily reach a desired location.

The hydrophilic coating 34 may also be applied to the inner surface of the microcatheter 20. Accordingly, the guide wire 10 may be easily retracted into and extended out of the microcatheter 20.

The microcatheter 20 may rotate within the blood vessel A. The microcatheter 20 may include a direction control mechanism. Accordingly, the forward direction of the medical instrument 1 may be adjusted so as not to penetrate the wall of a blood vessel, and the traveling direction of the guide wire 10 may be precisely controlled. For example, the microcatheter 20 may adjust the protrusion direction of the guide wire 10.

Particularly, the microcatheter 20 may include a first region 201 and a second region 202.

The first region 201 may form a tip portion of the microcatheter 20. The end 101 of the guide wire 10 may be retracted or extended through the first region 201. The first region 201 may have stiffness to smoothly advance within the blood vessel A.

The second region 202 may be connected to the first region 201 and may extend to the end portion of the microcatheter 20. In this case, the second region 202 may be connected to the first region 201 to form an angle. Accordingly, the first region 201 may control the direction of the guide wire 10.

Referring to FIG. 1(a), the first region 201 may be formed in a conical shape. Additionally, referring to FIG. 1(b), the first region 201 may be formed in a tapered shape. In this case, the first region 201 may be formed in a conical or tapered shape with a diameter decreasing toward the tip. Accordingly, the tip portion of the first region 201 may engage with the end 101 of the guide wire 10. The shape of the first region 201 and its coupling form with the end 101 may facilitate smooth passage of the medical instrument 1 through an occlusion.

Referring to FIG. 2, depending on the various profiles of the guide wire 10, the microcatheter 20 may also be provided with various profiles. For example, depending on the diameter of the guide wire 10, the diameter of the microcatheter 20 may also increase as illustrated in FIG. 2(a) or decrease as illustrated in FIG. 2(b).

Referring to FIG. 3, the microcatheter 20 may be further subdivided.

The first region 201 may include a safety zone 2011 and a flex zone 2012.

The safety zone 2011 may be a portion in which an angled shape formed in the first region 201 is maintained.

The flex zone 2012 may be a portion that is more flexible than the safety zone 2011 and may be a portion that bends when reaching an obstacle such as the wall of a blood vessel.

The second region 202 may include a transition zone 2021, a power zone 2022, a torque zone 2023, and a push zone 2024.

The transition zone 2021 may be a portion in which a transition from the first region 201 to the second region 202 occurs.

The torque zone 2023 may be a portion to which rotational force is applied to change the direction of the safety zone 2011.

The push zone 2024 may be a portion to which longitudinal force is applied to advance the microcatheter 20 within a blood vessel.

The steerable medical instrument 1 according to an embodiment may be inserted into the blood vessel A with a lesion and may safely fracture and pass through an occluded part using the guide wire 10.

The steerable medical instrument 1 according to an embodiment may be used, for example, for recanalization of a chronic total occlusion (CTO) lesion of a peripheral artery. However, the application of the steerable medical instrument 1 according to an embodiment is not limited thereto.

A dedicated device may be required to perform directional intravascular tracking (DIT). The steerable medical instrument 1 according to an embodiment may take the form of an assembly of the guide wire 10 and the microcatheter 20. The end of the guide wire 10 may have a complete J-curve shape and may have sufficient stiffness to pass through a CTO segment. Additionally, the guide wire 10 may freely advance or retract within the microcatheter 20. Accordingly, the guide wire 10 and the microcatheter 20 may move as a single unit, or each may move individually.

The microcatheter 20 may have a curved tip portion so that direction control is possible. The medical instrument 1, in which the guide wire 10 and the microcatheter 20 are assembled, may be safely tracked only within the blood vessel A without penetrating the wall of the blood vessel A.

The first step of performing DIT is to penetrate a proximal cap and then advance the guide wire 10 into an intraluminal or intraplaque space.

Because this process requires penetrating the proximal cap, it may be difficult to perform using the guide wire 10 having a complete J-curve shape. In this case, the proximal cap may be first penetrated using the guide wire 10 that is stiff and straight. After the microcatheter 20 is advanced along the guide wire 10, the end 101 of the guide wire 10 that is straight may deform into the end 101 having a complete J-curve shape, and a DIT process may be then performed. During tracking within the blood vessel A, when the end 101 of the medical instrument 1 is in contact with a hard lesion, such as the wall of the blood vessel A or a lesion, which is the calcification C, the end 101 may deflect, allowing an operator to change the direction of the medical instrument 1 and proceed. That is, the steerable medical instrument 1 according to an embodiment may be deflected and steered without the risk of perforating the wall of the blood vessel A.

Hereinafter, with reference to FIGS. 4 to 6, a method of controlling the steerable medical instrument 1 according to the embodiments described above is described in detail.

Referring to FIG. 4, a method of controlling the steerable medical instrument 1 according to an embodiment may include the following operations.

Operation S1 of providing, as a real-time image, an image of the blood vessel A captured using intravascular ultrasound (IVUS) or fluoroscopy

Operation S2 of inserting the steerable medical instrument 1 into the blood vessel A that is occluded

Operation S3 of extending the end 101 of the guide wire 10 out of the microcatheter 20 into the blood vessel A such that the end 101 of the guide wire 10 deforms into a loop shape

Operation S4 of advancing the microcatheter 20 along the blood vessel A while controlling the direction of the guide wire 10 based on the image of the blood vessel A

Operation S5 of retracting the end 101 of the guide wire 10 into the microcatheter 20, so that the end 101 of the guide wire 10, in an expanded form, may fracture the occlusion B when the end 101 of the guide wire 10 reaches the occlusion B within the blood vessel A

FIG. 5 illustrates DIT through a calcified CTO lesion via a soft proximal CTO cap. In this case, the steerable medical instrument 1 according to an embodiment may penetrate the lesion using the guide wire 10 that is straight after exploration.

Particularly, an image of the blood vessel A captured using IVUS or fluoroscopy may be provided as a real-time image to an operator. In this way, the steerable medical instrument 1 may be guided using IVUS or fluoroscopy, allowing the operator to perform a procedure while minimizing the risk of incision or perforation. The operator may insert the steerable medical instrument 1 formed by the coupling of the guide wire 10 and the microcatheter 20 into the blood vessel A that is occluded. The operator may control the guide wire 10 so that the end 101 of the guide wire 10 may be extended out of the tip of the microcatheter 20 into the blood vessel A. The guide wire 10, initially inserted straight within the microcatheter 20, may have the end 101 formed into a J-shaped form within the blood vessel A, as illustrated in FIG. 5(a).

The operator may use an IVUS or fluoroscopy device 40 to view an image within the blood vessel A in real time. Based on this image, the operator may advance the medical instrument 1 within the blood vessel A and rotate the microcatheter 20 to control the direction of the guide wire 10.

As illustrated in FIG. 5(b), when the end 101 reaches the occluded lesion B during advancement, the operator may control the guide wire 10 so that only a portion of the end 101 remains exposed while the rest is retracted into the microcatheter 20. The end 101 may deform into a straight shape within the microcatheter 20.

The operator may use the guide wire 10 in a straight shape to penetrate and fracture the occlusion B as shown in FIG. 5(c). In this case, the guide wire 10 may provide sufficient stiffness, as described above, to fracture or penetrate a lesion.

After fracturing the lesion, the operator may push the end 101 of the guide wire 10 out of the microcatheter 20, allowing the end 101 to return to a J-shaped form. In this state, the guide wire 10, together with a tapered tip 33 of the microcatheter 20, may smoothly pass through the lesion. In this case, the rotational function of the microcatheter 20 may allow precise adjustment of the direction of the guide wire 10, enabling safe passage through a portion with a lesion, which is the calcification C, as illustrated in FIGS. 5(d) to 5(h).

Additionally, the end 101 in a J-shaped form may flexibly move to safely pass through an occluded blood vessel while assisting in fracturing a hard portion of a lesion.

Particularly, during intravascular tracking with the steerable medical instrument 1, contact with a hard lesion, such as the lesion, which is the calcification C, may cause the end 101 in the J-shaped form to be deflected. In this case, the operator may rotate the microcatheter 20 to change the direction of the first region 201 and the end 101. As a result, the operator may control the deflection and direction of the medical instrument 1 without the need to perforate the wall of a blood vessel.

FIG. 6 illustrates an example of DIT through a calcified CTO lesion with a rigid proximal CTO cap. In this case, the steerable medical instrument 1 according to an embodiment may require penetration of a lesion with the guide wire 10 that is straight prior to exploration.

As in FIG. 5, an image of the blood vessel A captured using IVUS or fluoroscopy may be provided as a real-time image to the operator. In this way, the steerable medical instrument 1 may be guided using IVUS or fluoroscopy, allowing the operator to perform a procedure while minimizing the risk of incision or perforation. The operator may insert the steerable medical instrument 1 formed by the coupling of the guide wire 10 and the microcatheter 20 into the blood vessel A that is occluded.

As illustrated in FIG. 6(a), the steerable medical instrument 1 may reach the occluded lesion B in a state in which the end 101 of the guide wire 10 deforms into a straight shape. Using the stiffness of the end 101, the operator may advance the microcatheter 20 to penetrate and fracture the occlusion B, as illustrated in FIG. 6(b).

As illustrated in FIG. 6(c), after fracturing the occlusion B, the operator may retract the microcatheter 20. Additionally, the operator may extend the end 101 of the guide wire 10 out of the microcatheter 20 toward the blood vessel A, restoring the end 101 to a J-shaped form.

In this state, the operator may advance the steerable medical instrument 1, as illustrated in FIG. 6(d). The guide wire 10, together with the tapered tip 33 of the microcatheter 20, may then smoothly pass through the lesion. In this case, the rotational function of the microcatheter 20 may allow precise adjustment of the direction of the guide wire 10, enabling safe passage through a portion with a lesion, which is the calcification C, as illustrated in FIGS. 6(e) to 6(h).

Additionally, the end 101 in a J-shaped form may flexibly move to safely pass through an occluded blood vessel while assisting in fracturing a hard portion of a lesion.

Particularly, during intravascular tracking with the steerable medical instrument 1, contact with a hard lesion, such as the lesion, which is the calcification C, may cause the end 101 in the J-shaped form to be deflected. In this case, the operator may rotate the microcatheter 20 to change the direction of the first region 201 and the end 101. As a result, the operator may control the deflection and direction of the medical instrument 1 without the need to perforate the wall of a blood vessel.

According to the steerable medical instrument 1 according to an embodiment, the guide wire 10 may be coupled to the microcatheter 20, wherein the end 101 of the guide wire 10 has a J-shaped form, enabling precise directional control within the blood vessel A. This may allow an operator to easily steer the guide wire 10 in a desired direction, providing the advantage of safely advancing through a lesion without perforating the wall of the blood vessel A.

According to the steerable medical instrument 1 according to an embodiment, the guide wire 10 formed of a shape memory alloy may appropriately adjust flexibility and stiffness. Accordingly, even when passing through a hard lesion, which is the calcification C, the guide wire 10 may not deform or twist within the blood vessel A, enabling effective operation through a long and complex lesion.

According to the steerable medical instrument 1 according to an embodiment, an assembly of the guide wire 10 and the microcatheter 20 may be designed to safely pass through a lesion without damaging the wall of a blood vessel. This may significantly reduce the risk of the blood vessel A perforation or hematoma formation during a procedure, thereby enhancing procedural safety.

According to the steerable medical instrument 1 according to an embodiment, a variety of lesion types, including curved, calcified, and long occluded lesions, as well as short lesions may be handled, thereby significantly enhancing procedural versatility.

According to the steerable medical instrument 1 according to an embodiment, the success rate of recanalization of a chronic occlusive lesion of a peripheral artery may be increased, the workload of an operator may be reduced, and patient safety may be enhanced.

As described above, the embodiments of the present disclosure are described with specific details such as specific components, limited embodiments, and drawings; however, this is provided merely to aid in a more comprehensive understanding of the present disclosure, and the present disclosure is not limited to the embodiments described above. Those skilled in the art to which the present disclosure pertains may make various modifications and variations from this description. For example, suitable results may be achieved if the described techniques are performed in a different order, and/or if components in a described architecture, device, or the like are combined in a different manner, or replaced or supplemented by other components or their equivalents. Accordingly, the scope of the present disclosure is defined not by the detailed description, but by the claims and their equivalents, and all variations within the scope of the claims and their equivalents are to be construed as being included in the disclosure.

## Claims

1. A steerable medical instrument capable of moving along a blood vessel, the steerable medical instrument comprising:
a guide wire having an end formed in a loop shape; and
a microcatheter formed as a hollow tube and coupled to surround the guide wire,
wherein the guide wire is able to be retracted into or extended out of the microcatheter, and
the microcatheter rotates within a blood vessel to control a traveling direction of the guide wire.

2. The steerable medical instrument of claim 1,
wherein the guide wire is formed of a material having a shape memory property, and the end of the guide wire is restored to a J-shaped form within a blood vessel.

3. The steerable medical instrument of claim 2,
wherein the end of the guide wire has stiffness, and the stiffness of the end is greater than that of an occluded lesion of a blood vessel and less than that of a wall of the blood vessel.

4. The steerable medical instrument of claim 1, wherein
the microcatheter comprises:
a first region comprising a tip through which the end of the guide wire is retracted or extended; and
a second region connected to the first region to form an angle with respect to the first region, and
a direction in which the guide wire is extended is controlled according to a direction of the first region.

5. The steerable medical instrument of claim 4,
wherein the first region is formed in a conical or tapered shape having a diameter decreasing toward the tip, and the tip engages with the guide wire.

6. The steerable medical instrument of claim 1,
wherein an outer surface or an inner surface of the microcatheter is coated with a hydrophilic coating.

7. A method of controlling a steerable medical instrument formed by coupling of a guide wire and a microcatheter, wherein the guide wire has an end formed in a loop shape and the microcatheter is formed as a hollow tube, the method comprising:
providing, as a real-time image, a blood vessel image captured using intravascular ultrasound (IVUS) or fluoroscopy;
inserting the steerable medical instrument into a blood vessel that is occluded;
extending the end of the guide wire out of the microcatheter into the blood vessel such that the end of the guide wire deforms into a loop shape; and
advancing the microcatheter along the blood vessel while controlling a direction of the guide wire based on the blood vessel image.

8. The method of claim 7,
further comprising, after the advancing of the microcatheter along the blood vessel while controlling the direction of the guide wire based on the blood vessel image:
when the end of the guide wire reaches an occlusion within the blood vessel, retracting the end of the guide wire into the microcatheter, so that the end of the guide wire, in an expanded form, fractures the occlusion.
